(19) Europäisches Patentamt
European Patent Office
Office européen des brevets

(11) **EP 3 943 055 B1**

(12) **EUROPÄISCHE PATENTSCHRIFT**

(45) Veröffentlichungstag und Bekanntmachung des Hinweises auf die Patenterteilung:
**12.11.2025  Patentblatt 2025/46**

(51) Internationale Patentklassifikation (IPC):
*A61F 13/534* (2006.01)　　*A61F 13/532* (2006.01)
*A61F 13/533* (2006.01)

(21) Anmeldenummer: **20187625.7**

(22) Anmeldetag: **24.07.2020**

(52) Gemeinsame Patentklassifikation (CPC):
**A61F 13/532; A61F 13/533;** A61F 2013/53472;
A61F 2013/5349

(54) **SAUGKERNHÜLLE FÜR SUPERABSORBIERENDE SAUGKERNE VON HYGIENEARTIKELN**

ABSORBENT CORE SLEEVE FOR SUPERABSORBENT ABSORBENT CORES OF SANITARY ARTICLES

ENVELOPPE DU NOYAU ABSORBANT POUR NOYAUX SUPER-ABSORBANTS DES ARTICLES D'HYGIÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.01.2022  Patentblatt 2022/04**

(73) Patentinhaber: **Sandler AG**
**95126 Schwarzenbach/Saale (DE)**

(72) Erfinder:
• **Schuberth, Martin**
 **95236 Stammbach (DE)**
• **Bernhuber, Uwe**
 **95032 Hof (DE)**

(74) Vertreter: **Grünecker Patent- und Rechtsanwälte PartG mbB**
**Leopoldstraße 4**
**80802 München (DE)**

(56) Entgegenhaltungen:
**EP-A1- 3 338 749　　DE-T5- 112014 002 017**
**US-A1- 2015 173 968**

**Beschreibung**

Die vorliegende Erfindung betrifft einen Saugkern für Hygieneartikel mit einer Absorptionsmatrix, die einen großen Anteil superabsorbierender Partikel aufweist, und einer Saugkernhülle zum Einhüllen der Absorptionsmatrix, wobei die Saugkernhülle zumindest eine Oberseitenlage und eine Unterseitenlage aufweist und die Saugkernhülle mindestens einen vom Rand des Saugkerns beabstandeten Verbindungsbereich aufweist, in dem die Oberseitenlage und die Unterseitenlage miteinander verbunden sind. Darüber hinaus betrifft die Erfindung einen Hygieneartikel mit einem solchen Saugkern, die Verwendung eines Vliesverbunds als Saugkernhülle sowie ein Verfahren zur Herstellung eines Vliesverbunds für die Oberseitenlage der Saugkernhülle.

Hygieneartikel mit superabsorbierenden Saugkernen zur Aufnahme und Speicherung von Körperflüssigkeiten, wie beispielsweise Babywindeln, Hygieneeinlagen, Binden oder Inkontinenzunterwäsche, sollen bei möglichst geringer Dicke des Gesamtaufbaus ein möglichst hohes Speichervolumen für die aufzunehmenden Körperflüssigkeiten ermöglichen. Dabei soll die Körperflüssigkeiten so schnell wie möglich von der Haut weggeleitet werden, um Hautirritationen zu vermeiden und dem Träger ein sicheres und komfortables Gefühl zu geben. Dazu umfassen solche Hygieneartikel typischerweise mehrere Schichten oder Lagen, die verschiedene Funktionen bereitstellen und aufeinander abgestimmt sind. Üblicherweise bestehen derartige Hygieneartikel aus einer der Haut zugewandten Deckschicht (Topsheet), die für eine schnelle Aufnahme und Weiterleitung der Körperflüssigkeit sorgt, einer Aufnahme- und Verteillage (ADL - Acquisition-Distribution-Layer) zur raschen Aufnahme und Verteilung der auftreffenden Flüssigkeit über die gesamte Fläche des Hygieneprodukts, einem Saugkern zum Absorbieren und Immobilisieren der Körperflüssigkeit und einer flüssigkeitsdichten Abschlusslage (Backsheet), um ein Austreten der Körperflüssigkeit aus dem Hygieneartikel zu vermeiden.

Eine wesentliche Funktion des Saugkerns ist es, neben der Absorption und Immobilisierung der aufgenommenen Flüssigkeitsmenge diese für eine längere Zeit im Saugkern zu halten und eine Rücknässung zu minimieren. Viele im Handel erhältliche Hygieneartikel weisen einen Saugkern mit einer Absorptionsmatrix auf, die aus einer Mischung von zerkleinertem Zellstoff und superabsorbierenden Partikeln (SAP - Superabsorber-Polymer) besteht und in einer Saugkernhülle aufgenommen ist. Die Saugkernhülle besteht dabei aus einer der Aufnahme- und Verteillage zugewandten Oberseitenlage, durch die zumindest der Hauptteil der Körperflüssigkeit in den Saugkern eindringt, und einer der optionalen Abschlusslage zugewandten Unterseitenlage. Die Saugkernhülle dient neben der Aufnahme der Absorptionsmatrix auch für eine möglichst gleichmäßige Verteilung der Absorptionsmatrix im Saugkern und dafür, dass die Absorptionsmatrix an ihrem Platz im Saugkern bleibt. Die im Stand der Technik bekannten Saugkernhüllen zur Einkapselung der Absorptionsmatrix mit superabsorbierenden Partikeln bestehen üblicherweise aus einem dünnen Feinstfaservlies mit geringer Durchlässigkeit gegenüber dem superabsorbierenden Partikeln, beispielsweise aus einem einfachen Spinnvlies (Spunbond), einem schmelzgeblasenen Vlies (Meltblown) oder einem SMS-Vlies (Spunbond-Meltblown-Spunbond), das aus einer Kombination der Spinnvlies- und Schmelzblas-Verfahren hergestellt ist und thermisch mittels Kalandern verfestigt wird. Diese Vliesstoffe zur Herstellung einer herkömmlichen Saugkernhülle bestehen dabei üblicherweise aus thermoplastischen Polymeren.

Zur Reduzierung der Dicke des Saugkerns und damit zu einer Verbesserung des Tragekomforts für den Träger besteht im Stand der Technik die Tendenz in der Absorptionsmatrix den Anteil der superabsorbierenden Partikel gegenüber anderen Bestandteilen, insbesondere Zellstoffpartikeln, zu erhöhen, wobei auch Saugkerne mit ausschließlich superabsorbierenden Partikeln vorgeschlagen werden. Saugkerne mit einem großen Anteil an superabsorbierenden Partikeln neigen jedoch dazu, dass die in den Saugkern des Hygieneartikels eintretende Körperflüssigkeit nur örtlich begrenzt gebunden wird, wodurch sich eine große Flüssigkeitsmenge auf eine relativ kleine Fläche verteilt und nur die im Bereich dieser Fläche befindlichen superabsorbierenden Partikel zur Flüssigkeitsbindung aktiviert werden. Die außerhalb dieser Fläche befindlichen superabsorbierenden Partikel bleiben dagegen ungenutzt. Dadurch werden die von der Flüssigkeit benetzten superabsorbierenden Partikel sehr rasch zum Quellen gebracht, wodurch sich in der äußeren Zone des Saugkerns eine Gelschicht bildet, die das Eindringen weiterer Körperflüssigkeit in das Innere des Saugkerns verhindert. Dieser als "Gel-Blocking" bezeichnete Zustand verhindert eine gute Aufnahme weiterer spontan auftretender Flüssigkeitsmengen. Je größer in einer Absorptionsmatrix der Anteil an superabsorbierenden Partikeln ist, desto größer ist die Gefahr eines solchen Gel-Blockings. Deshalb wird im Stand der Technik versucht, bei Hygieneartikeln mit Saugkernen mit einem großen Anteil superabsorbierender Partikel eine spontan auftretend große Flüssigkeitsmenge vor dem Eindringen der Körperflüssigkeit in den Saugkern mittels der Aufnahme- und Verteillage und weiteren optionalen Zwischenlagen vor der Aufnahme durch den Saugkern auf eine möglichst große Fläche zu verteilen.

Zur Vermeidung oder Umgehung des Gel-Blockings, bzw. zur Besserung der Aufnahme und Verteilung der in den Saugkern eindringenden Körperflüssigkeiten, insbesondere bei einem schon zum Teil gequellten Saugkern, ist es bekannt, den Saugkern durch Kanäle in mehrere Bereiche zu unterteilen, wobei die Kanäle oberhalb des Saugkerns Flüssigkeit aus bereits blockierten Bereichen in Bereiche mit guter Absorptionsfähigkeit abführen. Aus der EP 2 717 823 A1 ist beispielsweise ein Saugkern für Hygieneartikel mit zwei im Wesentlichen in Längsrichtung verlaufenden Kanälen bekannt, die sowohl im trockenen als auch im nassen Zustand vorhanden sind. Sobald der Saugkern Flüssigkeit absorbiert und aufquillt, nehmen die Kanäle des Saugkerns eine dreidimensionale Form an. Hygieneartikel mit Kanälen ermöglichen eine verbesserte Passform sowie eine verbesserte Flüssigkeitsaufnahme und Flüssigkeitstransport während der gesamten Nutzung des Hygieneartikels. Aus der EP 1 827 335 A1 ist ein weiterer Hygieneartikel bekannt, bei dem der Saugkern einen großen Anteil an superabsorbierenden Partikeln aufweist und bei dem die Saugkernhülle mit mehreren Rillen oder Führungen versehen ist, durch die die Fluidaufnahme des Saugkerns verbessert wird.

Ein weiterer Hygieneartikel ist aus US2015/173968 bekannt.

Saugkerne für Hygieneartikel, die zumindest in einem nassen oder gequellten Zustand des absorbierenden Saugkerns dreidimensionale Kanäle zwischen der Oberseite des Saugkerns und einer darüber angeordneten Schicht, beispielsweise der Aufnahme- und Verteillage, ausbilden, können trotz der geringen Dicke des Saugkerns und seiner flächigen Struktur größere Mengen von eintretenden Körperflüssigkeiten schneller von der Oberfläche des Hygieneartikels abtransportieren und aufnehmen. Dabei wird die Flüssigkeitsmenge nicht sofort im Saugkern immobilisiert, sondern bewegt sich abhängig von der Lage des Hygieneartikels in den dreidimensionalen Kanalstrukturen. Insbesondere bei einer seitlichen Liegeposition folgt die nicht gebundene Flüssigkeit in den Kanälen der Schwerkraft, was bei einem bereits zum Teil gequellten Zustand des Saugkerns zu einem Auslaufen des Hygieneartikels führen kann.

Es ist daher die Aufgabe der vorliegenden Erfindung, die Aufnahme der Körperflüssigkeit in dem Saugkern eines Hygieneartikels zu verbessern und eine Bewegung nicht gebundener Körperflüssigkeiten in Kanälen an der Oberseite des Saugkerns zu reduzieren.

Diese Aufgabe wird erfindungsgemäß dadurch gelöst, dass zumindest die Oberseitenlage der Saugkernhülle für einen gattungsgemäßen Saugkern als ein Vliesverbund mit einer Fixierlage mit geringer Durchlässigkeit, um die Partikel in der Absorptionsmatrix zu halten, und einer Transferlage zur Aufnahme von Flüssigkeit, ausgebildet ist. Bei dem erfindungsgemäßen Saugkern für Hygieneartikel, insbesondere für Babywindeln und Inkontinenzunterwäsche, wird die eigentliche Funktion der Saugkernhülle, die Einkapselung der Absorptionsmatrix und Durchleitung der Flüssigkeit in den Saugkern, durch die Funktionen einer verbesserten Flüssigkeitsabsorption, Flüssigkeitsspeicherung und Flüssigkeitsweiterleitung erweitert. Dies wird durch den Einsatz eines Vliesverbunds für die Saugkernhülle erreicht, wobei nicht nur die der Aufnahme- und Verteillage, bzw. der Haut des Trägers, zugewandte Oberseitenlage, sondern auch die abgewandte Unterseitenlage als Vliesverbund ausgebildet sein kann. Dabei weist der Vliesverbund eine Fixierlage mit einer geringen Durchlässigkeit gegenüber den superabsorbierenden Partikeln und weiteren Partikeln der Absorptionsmatrix auf, um die Partikel in der Absorptionsmatrix zu halten und die Absorptionsmatrix an sich in dem Saugkern einzukapseln. Die Absorptionsmatrix des Saugkerns enthält neben einem großen Anteil superabsorbierender Partikel üblicherweise auch hydrophile Zellulosefasern und optional auch absorbierende Geliermaterialien, so dass die in den Saugkern eintretende Flüssigkeit auch bei einem großen Flüssigkeitsvolumen schnell gebunden werden kann. Dabei beträgt der Anteil der superabsorbierenden Partikel in der Absorptionsmatrix bevorzugt mindestens 35 %, insbesondere mindestens 80 % und optional bis zu 100 %. Die Oberseitenlage und die Unterseitenlage der Saugkernhülle sind sowohl am Rand des Saugkerns miteinander verbunden, um die Einkapselung des Saugkerns sicherzustellen, aber auch in mindestens einem vom Rand des Saugkerns beabstandeten Verbindungsbereich miteinander verbunden, beispielsweise mittels Klebstoff oder thermisch, so dass sich in diesem Bereich gegenüber dem restlichen Saugkern eine Vertiefung ausbildet. Bei einer länglichen Form des Vertiefungsbereichs bildet sich auf dem Saugkern ein Kanal aus, zumindest wenn die Absorptionsmatrix durch die Aufnahme von Flüssigkeit teilweise aufgequollen ist, der zur Weiterleitung der nicht gebundenen Flüssigkeit in einen nicht aufgequollenen Bereich des Saugkerns geeignet ist.

Die Fixierlage ist als ein Spinnvlies (Spunbond) oder ein schmelzgeblasenes Vlies (Meltblown) ausgebildet, insbesondere als ein SMS-Vlies (Spunbond-Meltblown-Spunbond) mit einem SMS-Lagenaufbau, und die Transferlage als ein Stapelfaservlies. Ein SMS-Vlies mit einem typischen Lagenaufbau, bei dem eine schmelzgeblasene Meltblown-Schicht in der Mitte zwischen den beiden Spinnvliesen (Spunbond) angeordnet ist und insbesondere durch Kalanderbonding mit den beiden Spinnvliesschichten verbunden ist, ist, wie aus dem Stand der Technik bekannt, mit einer geringen Luftdurchlässigkeit sehr gut für die Einkapselung der Partikel in der Absorptionsmatrix geeignet. Bevorzugt weist ein als Fixierlage eingesetztes SMS-Vlies ein Flächengewicht von $\leq 10$ g/m$^2$, insbesondere von $\leq 8$ g/m$^2$ auf. Alternativ kann die Fixierlage auch als ein einfaches Spinnvlies mit einer guten Festigkeit zur Einkapselung der Absorptionsmatrix oder als schmelzgeblasenes Vlies ausgebildet sein. Das als Transferlage eingesetzte Stapelfaservlies ermöglicht eine gute Aufnahme und Weiterleitung von Flüssigkeit und kann so die Verteilung von Flüssigkeit über den gesamten Saugkern verbessern. Dadurch kann trotz einer guten Einkapselung der Absorptionsmatrix die gesamte Oberfläche zumindest der Oberseitenlage der Saugkernhülle zum Flüssigkeitstransfer in den Saugkern genutzt werden kann.

Die Stapelfasern des Stapelfaservlieses sind als Grobfasern ausgebildet mit Faserfeinheiten im Bereich von 3,3 bis 12,0 dtex und einem zellulosischen Faseranteil von bis zu 45 %, bevorzugt zwischen 15 % bis 20 %. Eine solche Grobfaserstruktur des Stapelfaservlieses bietet ein sehr hohes freies Volumen pro Quadratmeter Fläche des Stapelfaservlieses, wodurch temporär eine sehr hohe Flüssigkeitsaufnahme erreicht wird. Durch den reduzierten Anteil an zellulosischen Fasern kann ein großer Anteil der absorbierten Flüssigkeitsmenge sofort in die Absorptionsmatrix des Saugkerns weitergegeben werden.

Eine besondere Ausgestaltung sieht vor, dass die Stapelfasern des Stapelfaservlieses als eine Kombination aus Feinfasern und Grobfasern ausgebildet sind, wobei die Feinfasern Faserfeinheiten im Bereich von 1,0 bis 3,0 dtex und einen zellulosischen Faseranteil von 50 % bis 100 % und die Grobfasern Faserfeinheiten im Bereich von 3,3 bis 12,0 dtex und einen zellulosischen Faseranteil von bis zu 45 %, bevorzugt zwischen 15 % bis 20 %, aufweisen. Ein Stapelfaservlies mit einer Kombination aus Feinfasern und Grobfasern ermöglicht sowohl, dass eine hohe körperseitige Flüssigkeitsaufnahme des Stapelfaservlieses von der hohen kapillaren Flüssigkeitsabsorption über die Feinfasern und das große freie Volumen der Grobfasern unterstützt wird. Dabei kann eine der Absorptionsmatrix zugewandte Fixierlage aus einem SMS-Vlies die Entwässerung des eingenadelten Feinfaservlieses in Richtung des Saugkerns unterstützen, wodurch sich eine besonders schnelle und großflächige Verteilung der Flüssigkeit im Saugkern ergibt. In der Anwendung eines solchen Stapelfaservlieses in einem Vliesverbund für die Oberseitenlage einer Saugkernhülle für Hygieneartikel sind die Feinfasern auf der Seite der Fixierlage angeordnet und die Grobfasern der Haut eines Trägers des Hygieneartikels zugewandt.

Bevorzugt kann das Stapelfaservlies mittels eines Spunlaceprozesses hergestellt sein. Beim Spunlaceprozess werden Wasserstrahlen mit Hochdruck auf einen Faserflor geschossen, um das entstehende Stapelfaservlies zu verdichten. Die aus den Düsen mit sehr geringem Durchmesser austretenden Wasserstrahlen unter hohem Druck führen dazu, dass die Stapelfasern des Stapelfaservlieses sich miteinander verbinden und verfestigt werden. Dabei wirken sich die durch den Wasserstrahlprozess entstehenden Kapillare im Stapelfaservlies zusätzlich vorteilhaft auf eine kontinuierliche Flüssigkeitsaufnahme in Richtung zum Saugkern hin aus, ohne die ursprünglichen Absorptionseigenschaften des Stapelfaservlieses zu behindern. Durch den Spunlaceprozess kann in dem Stapelfaservlies ein erhöhtes Porenvolumen realisiert werden.

Eine vorteilhafte Ausgestaltung sieht vor, dass das Stapelfaservlies in ein als Fixiervlies verwendetes, vorverfestigtes SMS-Vlies eingenadelt ist. Das Vernadeln des Stapelfaservlieses mit einem als Fixiervlies verwendetes vorverfestigten SMS-Vlieses ist eine einfache und sichere mechanische Vliesverfestigung zur Verbindung der beiden Vlieslagen. Dabei kann die Einbindung der Stapelfasern in das SMS-Vlies die Grenzflächeneffekte zwischen dem SMS-Vlies und dem Stapelfaservlies minieren und den Füssigkeitstransfer zwischen den beiden Vliesen verbessern.

Günstigerweise kann das Stapelfaservlies eine Faseroberfläche von größer als 10 m², bevorzugt von größer als 12 m², pro 1 m² Oberfläche des Stapelfaservlieses bei planliegender Vliesfläche aufweisen. In Abhängigkeit der Vliesfläche gilt je größer die Faseroberfläche des Stapelfaservlieses in der dem Saugkern abgewandten Oberfläche der Saugkernhülle ist, desto größer ist bei gleichen Faserfeinheiten der vorhandene Hohlraum zur Aufnahme von Flüssigkeit im Stapelfaservlies. Des Weiteren kann mittels der Größe der Faseroberfläche im Stapelfaservlies eine Unterscheidung zwischen Feinfaservliesen und Grobfaservliesen getroffen werden.

Ein Ausführungsbeispiel sieht vor, dass das Stapelfaservlies der Oberseitenlage ein freies Volumen von größer als 300.000 mm³, bevorzugt größer als 450.000 mm³, pro 1 m² Oberfläche bei planliegender Vliesfläche aufweist. Das freie Volumen des Stapelfaservlieses an der dem Saugkern abgewandten Oberfläche der Saugkernhülle ist das Maß für den vorhandenen Hohlraum des Stapelfaservlieses. Je größer das freie Volumen im Stapelfaservlies, desto mehr Flüssigkeit kann von dem Vlies aufgenommen und eingelagert werden.

Eine günstige Ausbildung sieht vor, dass zumindest die Oberseitenlage der Saugkernhöhle eine Dicke von größer als 0,35 mm aufweist, bevorzugt eine Dicke zwischen 0,5 mm und 1,2 mm aufweist. Mit steigender Dicke der Oberseitenlage steigt mit dem Volumen der Oberseitenlage auch das freie Volumen bzw. der vorhandene Hohlraum in der Oberseitenlage der Saugkernhülle und damit auch die Fähigkeit, Flüssigkeiten aufzunehmen und an die Absorptionsmatrix im Saugkern zu verteilen.

Für eine sichere Einkapselung der Absorptionsmatrix durch die Saugkernhülle kann zumindest die Oberseitenlage der Saugkernhülle bei einem Prüfdruck von 200 Pa eine Luftdurchlässigkeit von maximal 3.500 l/m²/s aufweisen, bevorzugt von maximal 3.000 l/m²/s aufweisen. Die Luftdurchlässigkeit ist der typische Wert zur Beurteilung der Dichtigkeit von Vliesstoffen, hier der Oberseitenlage der Saugkernhülle, die zur Vermeidung des Austretens von superabsorbierender Partikel eine ausreichende Dichtigkeit aufweisen muss.

In einer zweckmäßigen Form kann zumindest die Oberseitenlage der Saugkernhülle bei einem Wicking-Versuch nach 300 Sekunden eine Steighöhe von mindestens 10 mm, insbesondere mindestens 20 mm, aufweisen. Die Bestimmung der Steighöhe in einem Wicking-Versuch beschreibt das durch die Kapillarkräfte im Vlies gegen die Schwerkraft hervorgerufene Einzugsverhalten von Flüssigkeiten. Je größer die Steighöhe, desto größer sind die in Kapillarkanälen im Vlies erzeugten Kapillarkräfte und der entsprechende Dochteffekt auf die außerhalb der Oberseitenlage der Saugkernhülle vorhandene Flüssigkeit. Hohe Kapillarkräfte ermöglichen eine gute Flüssigkeitsaufnahme und -verteilung in der Oberseitenlage, wobei die Kapillarkräfte in Abhängigkeit der Benetzungseigenschaften der Faseroberfläche und der Größe der Kapillaren bei gleichen Steighöhen unterschiedlich sind.

Bevorzugt kann zumindest die Oberseitenlage der Saugkernhülle ein Gesamtporenvolumen von mindestens 0,90 (90 Vol.-%), insbesondere von 0,925 (92,5 Vol.-%), aufweisen. Ein hohes Gesamtporenvolumen ermöglicht eine hohe maximale Wasseraufnahme. Dabei kann die maximale Wasseraufnahme zumindest der Oberseitenlage der Saugkernhülle größer 7 g/g Oberseitenlage sein, bevorzugt zwischen 7 g/g und 12 g/g betragen, oder größer als 300 ml/m² Oberseitenlage sein, bevorzugt zwischen 300 ml/m² und 800 ml/m², betragen. Je größer die maximale Wasseraufnahme der Oberseitenlage ist, desto größer ist die Flüssigkeitsmenge, die von der Oberseitenlage ohne Druckbelastung absorbiert werden kann. Dabei ist die Angabe der Menge der Flüssigkeit, die pro Fläche der Oberseitenlage absorbiert werden kann, eine gute Einschätzung für die Menge der Flüssigkeit, die von der Oberseitenlage aufgenommen und immobilisiert werden kann.

In einer besonderen Ausführungsform kann der Run-Off-Anteil für zumindest die Oberseitenlage der Saugkernhülle bei einem Run-Off-Versuch in Anlehnung an WSP 80.9 kleiner als 50 % sein. Die WSP 80.9 ist ein Standard der EDANA (European Disposables and Nonwovens Associations). Bei einem in Anlehnung an WSP 80.9 durchgeführten Run-Off-Versuch wird innerhalb von 4 ± 0,1 Sekunden 25 ml einer 0,9 % NaCl-Lösung auf die Oberseitenlage der Saugkernhülle aufgebracht, wobei die Saugkernhülle im Gegensatz zu einem offiziellen Run-Off-Versuch nach WSP 80.9 die Oberseitenlage in einem Winkel von 30° angeordnet ist und nicht in einem Winkel 25°. Dies bedeutet, dass die in diesem Run-Off-Versuch in Anlehnung an WSP 80.9 erzielten Ergebnisse besser zu beurteilen sind als die aus einem Versuch nach WSP 80.9. Der Run-Off-Versuch ermöglicht eine Aussage über die Fähigkeit der Oberseitenlage eine definierte Menge an einer Urinersatzflüssigkeit an die darunterliegende Lage weiterzuleiten, während durch den Versuchsaufbau die Schräglage des Hygieneartikels simuliert wird. Je geringer der prozentuale Wert ist, desto besser ist die Aufnahme bzw. die Weiterleitung der Flüssigkeit in die Oberseitenlage und in die darunterliegende Absorptionsmatrix des Saugkerns. Des Weiteren sollte bei diesem Versuch die absolute Menge der nicht von der Oberseitenlage absorbierten Urinersatzflüssigkeit möglichst gering sein, da die nicht von der Oberseitenlage oder der Absorptionsmatrix aufgenommene Urinersatzflüssigkeit als ungebundene Flüssigkeit in einem Hygieneartikel zum Auslaufen des Hygieneartikels führen kann.

Vorteilhafterweise kann die Strike-Through-Zeit für zumindest die Oberseitenlage der Saugkernhülle bei einem Strike-Through-Versuch kleiner als 3 Sekunden sein, bevorzugt kleiner als 1,5 Sekunden sein. Bei einem Strike-Through-Versuch wird eine definierte Flüssigkeitsmenge punktuell in die Oberseitenlage der Saugkernhülle eingebracht und die Zeit gemessen, bis die Flüssigkeitsmenge von der Oberseitenlage, bevorzugt dem Stapelfaservlies der Transferlage, aufgenommen wird. Je kürzer die Strike-Through-Zeit, desto besser funktioniert die Aufnahme von Flüssigkeit in die Oberseitenlage.

Des Weiteren bezieht sich die vorliegende Erfindung auf einen Hygieneartikel mit einer optionalen Deckschicht (Topsheet), einer Aufnahme- und Verteillage (ADL), einem Saugkern für Hygieneartikel nach einer der oben beschriebenen Ausführungsformen und einer optionalen Abschlusslage (Backsheet), wobei der Saugkern eine Saugkernhülle mit einer Oberseitenlage und einer Unterseitenlage aufweist, die mindestens einen vom Rand des Saugkerns beabstandeter Bereich aufweist, in dem die Oberseitenlage und die Unterseitenlage miteinander verbunden sind und der gegenüber der Aufnahme- und Verteillage einen üblicherweise länglichen Kanal ausbildet, wenn die Absorptionsmatrix durch eine Aufnahme von Flüssigkeit aufquillt. Ein solcher Hygieneartikel ermöglicht eine verbesserte Flüssigkeitsaufnahme, der in den Kanälen zwischen der Saugkernhülle und der Aufnahme- und Verteillage vorhandenen freien Flüssigkeit und dessen Verteilung im Saugkern. Dadurch wird bei Hygieneartikeln mit modernem Saugkern deren Absorptionsmatrix einen großen Anteil von superabsorbierender Partikel aufweist und die im aufgequollenen Zustand Kanäle gegenüber der Aufnahme- und Verteillage ausbilden, ein Auslaufen des Hygieneprodukts verhindert.

Darüber hinaus bezieht sich die Erfindung auf die Verwendung eines Vliesverbunds mit einer Fixierlage und einer Transferlage als Saugkernhülle eines Saugkerns für Hygieneartikel, insbesondere für die der Haut eines Trägers zugewandte Oberseitenlage, wobei der Saugkern eine Absorptionsmatrix mit einem großen Anteil superabsorbierender Partikel umfasst, wobei die Saugkernhülle zum Einhüllen der Absorptionsmatrix ausgebildet ist, die Fixierlage eine geringe Durchlässigkeit aufweist, um die Partikel in der Absorptionsmatrix zu halten, und die Transferlage zur Aufnahme von Flüssigkeit geeignet ist. Ein derartiger Vliesverbund als Saugkernhülle verbessert die Aufnahme von Flüssigkeit und deren Verteilung im Saugkern, insbesondere bei einer bereits zum Teil aufgequollenen Absorptionsmatrix.

Ferner umfasst die Erfindung ein Verfahren zur Herstellung eines Vliesverbunds für eine Oberseitenlage einer Saugkernhülle eines Saugkerns für Hygieneartikel, mit einer Fixierlage aus einem vorverfestigten SMS-Vlies und einer Transferlage aus Stapelfasern, wobei die Transferlage aus Stapelfasern in das vorverfestigte SMS-Vlies eingenadelt wird. Bevorzugt kann die Transferlage als ein Stapelfaservlies ausgebildet sein, das mittels eines Spunlaceprozesses hergestellt wird.

Im Folgenden werden nichteinschränkende Ausführungsbeispiele der vorliegenden Erfindung anhand beispielhafter Zeichnungen näher erläutert. Es zeigen:

Fig. 1  zeigt eine Draufsicht auf einen erfindungsgemäßen Saugkern für Hygieneartikel,

Fig. 2  zeigt einen Schnitt durch den Saugkern aus Fig. 1 entlang der Linie II-II,

Fig. 3  zeigt einen Schnitt durch einen Hygieneartikel mit einem erfindungsgemäßen Saugkern nach Fig. 1 und Fig. 2,

Fig. 4  zeigt einen Schnitt durch den Hygieneartikel aus Fig. 3 in einem zumindest teilweise aufgequollenen Zustand, und

Fig. 5    zeigt einen Schnitt durch die Oberschicht einer Saugkernhülle für einen Saugkern nach Fig.1 und Fig. 2.

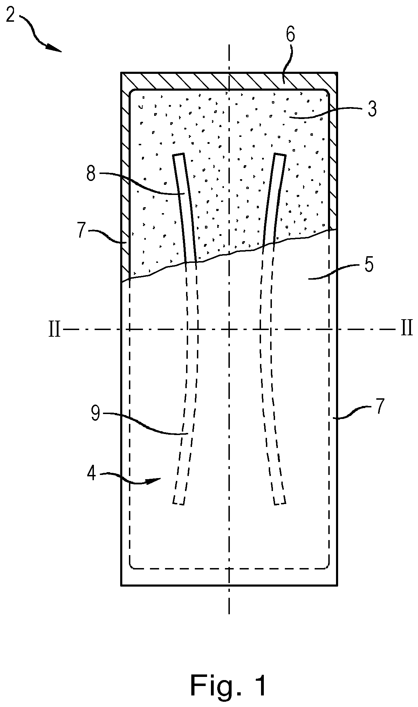

Der in Fig. 1 schematisch dargestellte beispielhafte Saugkern 2 für einen Hygieneartikel 1, wie beispielsweise Babywindel, Hygieneeinlage, Binden oder Inkontinenzunterwäsche, umfasst eine Absorptionsmatrix 3 aus superabsorbierenden Partikeln (SAP) und gegebenenfalls weiteren Partikeln, wie beispielsweise Zellstoff oder Geliermittel, und eine Saugkernhülle 4, die die Absorptionsmatrix 3 umschließt. Die Saugkernhülle 4 besteht dabei aus einer Oberseitenlage 5 und einer Unterseitenlage 6, die die Absorptionsmatrix 3 auf beiden Seiten umgeben und am Rand 7 der Absorptionsmatrix 3 miteinander verbunden sind, beispielsweise mittels Kleben oder mittels eines thermischen Verbindungsverfahrens. Die Saugkernhülle 4 dient neben der Einkapselung der Absorptionsmatrix 3, d. h. der Vermeidung eines Durchtritts der Partikel der Absorptionsmatrix 3 durch die Saugkernhülle 4, zum Transport von Flüssigkeit durch die Saugkernhülle 4 hindurch in die Absorptionsmatrix 3. In der erfindungsgemäßen Ausgestaltung des Saugkerns 2 ermöglicht die Saugkernhülle 4 neben einem wesentlichen senkrechten Transfer der Flüssigkeit durch die Saugkernhülle 4 in die Absorptionsmatrix 3 zusätzlich eine verbesserte Flüssigkeitsabsorption, eine Speicherung der Flüssigkeit in der Saugkernhülle 4 und die Weiterleitung der Flüssigkeit innerhalb der Saugkernhülle 4 in andere Bereiche des Saugkerns 2. Die Oberseitenlage 5 der Saugkernhülle 4 ist dabei der Haut eines Trägers zugewandt.

Der Saugkern 2 umfasst weiter mindestens einen vom Rand 7 des Saugkerns 2 beabstandeten Verbindungsbereich 8 auf, in dem die Oberseitenlage 5 und die Unterseitenlage 6 der Saugkernhülle 4 miteinander verbunden sind, beispielsweise mittels eines thermischen Verfahrens oder eines Klebstoffs. In diesem Verbindungsbereich 8 ist im Bereich der Absorptionsmatrix 3 zwischen der Oberseitenlage 5 und der Unterseitenlage 6 im Wesentlichen keine absorbierenden Partikel der Absorptionsmatrix 3, bzw. im Wesentlichen nur inaktivierte Partikel der Absorptionsmatrix 3, so dass sich in dem Verbindungsbereich 8 gegenüber dem restlichen Saugkern 2 einer oder mehrere Kanäle 9 ausbilden. Diese Kanäle 9 dienen dazu, eine in den Hygieneartikel 1 eintretende Flüssigkeit entlang der Länge der Kanäle 9 über den Saugkern 2 zu verteilen.

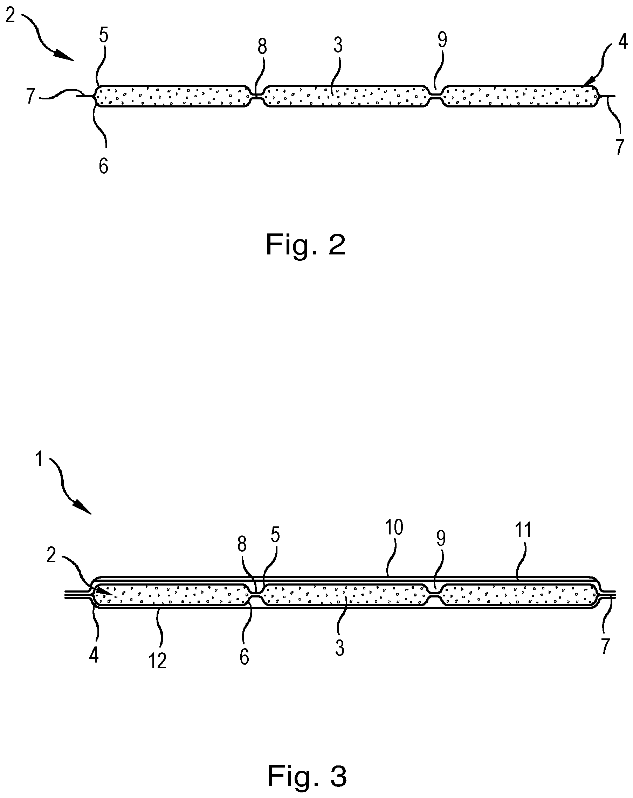

Fig. 2 zeigt einen Schnitt durch den erfindungsgemäßen Saugkern 2 aus Fig. 1 entlang der Linie II-II. Neben den am Rand des Saugkerns 2 miteinander verbundenen Oberseitenlage 5 und Unterseitenlage 6 der Saugkernhülle 4 sind hier deutlich auch die Verbindungsbereiche 8 zwischen der Oberseitenlage 5 und der Unterseitenlage 6 im Abstand zum Rand 7 der Saugkernhülle 4 zu erkennen.

Die Schnittansicht durch einen erfindungsgemäßen Hygieneartikel 1 in Fig. 3 zeigt die Anordnung des in den Fig. 1 und 2 gezeigten Saugkerns 2 in einem Hygieneartikel 1, beispielsweise in Babywindeln, Hygieneeinlagen, Binden oder Inkontinenzunterwäsche. Der Hygieneartikel 1 umfasst eine optionale Deckschicht 10 (Topsheet), die mit der Haut des Trägers direkt in Kontakt ist. Die Deckschicht 10 ist vorzugsweise nachgiebig, weich und nicht reizend für die Haut des Trägers. Dabei ist die Deckschicht 10 besonders flüssigkeitsdurchlässig, so dass Körperflüssigkeit leicht durch die Deckschicht 10 hindurchdringen kann. Zwischen der Deckschicht 10 und dem Saugkern 2 ist weiter eine Aufnahme- und Verteillage 11 (ADL) vorgesehen, die zu einer raschen Aufnahme und Übergabe der auftretenden Flüssigkeit an den Saugkern 2 dient. Die Aufnahme- und Verteillage 11 erzeugt einen Abstand zwischen dem Saugkern 2 und der Deckschicht 10 und reduziert so weiter die Gefahren der Rücknässung an den Träger. Als unterer Abschluss des Hygieneartikels 1 ist eine Abschlusslage 12 (Backsheet) vorgesehen. Die Abschlusslage 12 besteht üblicherweise aus einem wasserdichten Material, das atmungsaktiv oder dampfdicht ausgebildet sein kann. Der Saugkern 2 ist dabei zwischen der Abschlusslage 12 und der Aufnahme- und Verteillage 11 positioniert, wobei die Unterseitenlage 6 der Saugkernhülle 4 zur Abschlusslage 12 hin positioniert ist. Die Abschlusslage 12 kann ein Austreten der im Saugkern 2 absorbierten Flüssigkeit nach außen hin verhindern. Die Abschlusslage 12 bildet den größten Teil der äußeren Oberfläche des Hygieneartikels 1 aus, wenn dieser vom Träger getragen wird und verhindert so ein Verschmutzen von Bettwäsche oder Unterwäsche.

Der zwischen der Abschlusslage 12 und der Aufnahme- und Verteillage 11 positionierte Saugkern 2 stellt den größten Teil der Absorptionskapazität des Hygieneartikels 1 bereit. Die von der Saugkernhülle 4 eingekapselte Absorptionsmatrix 3 des Saugkerns 2 umfasst als flüssigkeitsabsorbierende Partikel einen großen Anteil an superabsorbierenden Partikeln (SAP), sowie weitere absorbierende Partikel, wie Zellstoff, synthetische Fasern und Geliermaterialien. Der Anteil an superabsorbierenden Partikeln kann dabei mindestens 50 %, bevorzugt mindestens 80 % und optional bis zu 100 % des Gesamtgewichts der absorbierenden Partikel der Absorptionsmatrix 3 sein. Dies ermöglicht im Vergleich zu herkömmlichen Saugkernen mit einem hohen Anteil an Zellstoffpartikeln einen relativ dünnen erfindungsgemäßen Saugkern 2, der neben einer erhöhten Flüssigkeitsaufnahme eine verbesserte Passform und einen höheren Tragekomfort des Hygieneartikels 1 ermöglicht.

Die Deckschicht 10 und die Abschlusslage 12 sowie optional auch die Aufnahme- und Verteillage 11 können am Rand 7 des Saugkerns 2 mit der Saugkernhülle 4 verbunden sein, um den Saugkern 2 sicher in dem Hygieneartikel 1 zu positionieren.

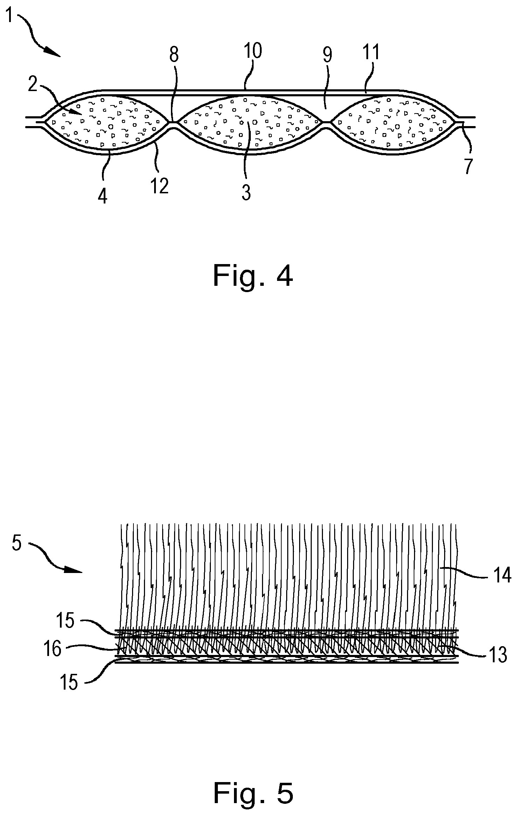

Fig. 4 zeigt den in Fig. 3 beschriebenen Hygieneartikel 1 in einem Zustand, in dem die Absorptionsmatrix 3 zumindest bereits teilweise durch bereits aufgenommene Flüssigkeit aufgequollen ist. In diesen zumindest teilweise aufgequollenen Zustand der Absorptionsmatrix 3 bilden sich zwischen der Oberseitenlage 5 der Saugkernhülle 4 und der Aufnahme- und Verteillage 11 große Kanäle 9 aus, über die die in den Hygieneartikel 1 eingetretenen Körperflüssigkeiten schnell zu anderen Bereichen des Saugkerns 2 transportiert werden können, um dort von der Absorptionsmatrix 3 des Saugkerns 2 absorbiert zu werden. Im Gegensatz zu herkömmlichen Saugkernen, bei denen in einer seitlichen Liegeposition des Trägers die nicht gebundene Flüssigkeit in den Kanälen aufgrund der Schwerkraft zu einem Auflaufen des Hygieneartikels führen kann, wird bei einem erfindungsgemäßen Saugkern 2 die nicht gebundene Flüssigkeit in den Kanälen 9 von der Oberseitenlage 5 der Saugkernhülle 4 trotz eines möglichen örtlichen Gel-Blockings schnell absorbiert, gespeichert, in der Oberseitenlage 5 weitergeleitet und dann in einem nicht blockierten Bereich der Absorptionsmatrix 3 an die Absorptionsmatrix 3 abgegeben.

In Fig. 5 ist beispielhaft der Aufbau einer Oberseitenlage 5 der Saugkernhülle 4 dargestellt. Die Oberseitenlage 5 besteht aus einem Vliesverbund mit einer Fixierlage 13 und einer Transferlage 14, wobei die Transferlage 14 in einem erfindungsgemäßen Hygieneartikel 1 nach Fig. 3 und Fig. 4 der Aufnahme- und Verteillage 11 zugewandt ist und die Fixierlage 13 der Absorptionsmatrix 3 des Saugkerns 2 zugewandt ist. Die Fixierlage 13 ist dabei bevorzugt als ein SMS-Vlies ausgebildet mit einem typischen SMS-Lagenaufbau, in den zwischen zwei Spinnvliesen 15 ein schmelzgeblasenes Meltblown-Vlies 16 angeordnet und durch Kalanderbonding mit den Spinnvliesen 15 verbunden ist. Diese Fixierlage 13 weist eine geringe Durchlässigkeit auf und dient daher im Wesentlichen dazu, die Partikel der Absorptionsmatrix 3 im Saugkern 2 zu halten. Alternativ kann diese Fixierlage 13 auch aus einem einfachen Spinnvlies 15 oder einem einfachen schmelzgeblasenes Meltblown-Vlies 16 ausgebildet sein, wodurch ein etwas einfacherer Aufbau der Oberseitenlage 5 mit einer einschichtigen Fixierlage 13 erreicht wird, sofern in Verbindung mit der Transferlage 14 eine insgesamt geringe Durchlässigkeit der Oberseitenlage 5 erreicht wird. Dem gegenüber dient die mit der Fixierlage 13 verbundene Transferlage 14 dazu, freie Flüssigkeit möglichst schnell aufzunehmen, in der Transferlage 14 zu verteilen und zwischen zu speichern sowie über die Fixierlage 13 an die Absorptionsmatrix 3 abzugeben. Dabei ist die bevorzugt als Stapelfaservlies ausgebildete Transferlage 14 in die Fixierlage 13 eingenadelt, wodurch Grenzflächeneffekte am oberen Spinnvlies 15 der Fixierlage 13 minimiert und der Flüssigkeitstransfer von der Transferlage 14 in die Fixierlage 13 und schlussendlich in die Absorptionsmatrix 3 verbessert werden kann.

Im Folgenden werden verschiedene Ausführungsformen der Oberseitenlage 5 der Saugkernhülle 4 für einen erfindungsgemäßen Saugkern 2 näher erläutert und deren Eigenschaften beschrieben. Aus Vergleichsgründen ist auch ein für herkömmliche Saugkernhüllen eingesetztes SMS-Vlies aufgeführt.

Neben dem zum Vergleich aufgeführten SMS-Vlies gemäß dem Stand der Technik wird in der folgenden Tabelle der Aufbau von fünf unterschiedlichen Verbundvliesen für die Oberseitenlage 5 der Saugkernhülle 4 eines erfindungsgemäßen Saugkerns 2 beschrieben.

Tabelle 1: Aufbau der in den Versuchen eingesetzten Verbundvliese.

| Vlies-Nr. | Bezeichnung | Fixierlage | Transferlage |
|-----------|-------------|------------|--------------|
| 1 | Vergleichsvlies | SMS-Vlies aus Polypropylen, 8 g/m$^2$ | - |
| 2 | Feinfaser-mischung 1 | SMS-Vlies aus Polypropylen, 8 g/m$^2$ | Stapelfaservlies aus Viskose 1,7 dtex, 22 g/m$^2$ |
| 3 | Feinfaser-mischung 2 | SMS-Vlies aus Polypropylen, 8 g/m$^2$ | Stapelfaservlies aus Viskose 1,7 dtex, 47 g/m$^2$ |
| 4 | Grobfasermischung 1 | SMS-Vlies aus Polypropylen, 8 g/m$^2$ | Stapelfaservlies aus 40 % Polyester 10 dtex, 40 % Polyester/CoPolyester 4,4 dtex, 20 % Viskose 1,7dtex, 60 g/m$^2$ |
| 5 | Grobfasermischung 2 | SMS-Vlies aus Polypropylen, 8 g/m$^2$ | Stapelfaservlies aus 40% Polyester 10 dtex, 32,5 % Polyester/CoPolyester 4,4 dtex, 27,5 % Viskose 1,7dtex, 60 g/m$^2$ |
| 6 | graduelle Mischung | SMS-Vlies aus Polypropylen, 8 g/m$^2$ | Stapelfaservlies aus Grobfaser mit 60% Polyester 3,3 dtex, 15% Polyester /CoPolyester 4,4 dtex, 25% Viskose 1,7dtex, und Feinfaser aus Viskose 1,7 dtex, 60 g/m$^2$ |

Vlies-Nr. 2 und 3 sind nicht erfindungsgemäße Beispiele.

Zur Bewertung der Eigenschaften der verschiedenen Verbundvliese und des Vergleichsvlieses aus dem Stand der Technik für den Einsatz als Oberseitenlage 5 der Saugkernhülle 4 eines erfindungsgemäßen Saugkerns 2 wurden die Vliese verschiedenen Tests unterzogen, die im Folgenden im Detail beschrieben sind.

Maximale Wasseraufnahme

Über die maximale Wasseraufnahme wird ausgedrückt, welche Flüssigkeitsmenge von einem der oben aufgeführten Vliese ohne Druckbelastung absorbiert werden kann. Durch die Angabe der maximalen Wasseraufnahme in Prozent und in Gramm pro Gramm des Vlieses kann die Fähigkeit eines Materials beschrieben werden, freie Flüssigkeit zu immobilisieren. Um besser einschätzen zu können welche Menge an Flüssigkeit pro Fläche der eingesetzten Oberseitenlage aufgenommen werden kann, empfiehlt sich dabei eine Umrechnung auf die aufgenommene Flüssigkeit in Milliliter bezogen auf einen Quadratmeter Vlies.

Gemäß dem Einsatzzweck als Oberseitenlage 5 einer Saugkernhülle 4 ist eine hohe Wasseraufnahme vorteilhaft, da das Vlies dann die Eigenschaft besitzt größere Mengen an Flüssigkeit zu immobilisieren.

Tabelle 2: Maximale Wasseraufnahme in Prozent, in Gramm pro Gramm Vlies und in Milliliter pro Quadratmeter Vlies.

| Vlies-Nr. | Max. WA [%] | Max. WA [g/g] | Max. WA [ml/m²] |
|---|---|---|---|
| 1 | 57 | 0,57 | 4,56 |
| 2 | 1110 | 11,10 | 333 |
| 3 | 742 | 7,42 | 482,3 |
| 4 | 1180 | 11,80 | 708 |
| 5 | 808 | 8,08 | 633,6 |
| 6 | 1056 | 10,56 | 404 |

Run-off

Der durchgeführte Run-off Versuch ist angelehnt an den Standard WSP 80.9 der EDANA (European Disposables and Nonwovens Association). Dabei werden innerhalb von 4 +/-0,1 Sekunden 25 ml einer 0,9% NaCl-Lösung auf ein geneigtes Flies aufgebracht. Anders als in dem Standard WSP 80.9 wurde für diesen Test das Vlies mit einem Winkel von 30° anstelle von 25° geneigt.

Der Run-off Versuch liefert eine Aussage über die Fähigkeit eines Materials eine definierte Menge an Urinersatzflüssigkeit an die darunterliegende Lage weiter zu leiten, während durch den Versuchsaufbau eine Schräglage eines Hygieneartikels 1 simuliert wird. Je geringer der prozentuale Wert ist, desto besser ist die Aufnahme und die Weiterleitung der Flüssigkeit in das Material sowie in die darunterliegende Absorptionsmatrix 3 des Saugkerns 2. Zusätzlich wird noch die absolute Menge der nicht aufgenommenen Flüssigkeit (Run-offs) in Milliliter angegeben. Hier gilt, je höher die absolute Menge des Run-offs ist, desto weniger Flüssigkeit wird vom Vlies und dem darunterliegenden Saugkern 2 absorbiert. Dies ist als negativ einzustufen, da ungebundene Flüssigkeit in einem Hygieneartikel 1 über die freie Bewegung in den Kanälen 9 zum Auslaufen des Hygieneartikels 1 führen kann, was einen Qualitätsmangel darstellt.

Die Größen "Run-off Aufnahme Papier" und "Run-off Aufnahme Vlies" zeigen ob sich die aufgenommene Flüssigkeit direkt nach der Absorption stärker im Vlies oder stärker im Saugkern 2 einlagert. Tendenziell ist es von Vorteil, wenn sich mehr Flüssigkeit im Papier, also im Saugkern 2, einlagert, denn dort soll die Flüssigkeit final gespeichert werden. Dies reduziert zusätzlich die Wahrscheinlichkeit der Rücknässung des Hygieneartikels 1.

In modernen Konstruktionen von Hygieneartikeln 1 mit Flüssigkeitskanälen 9 (z.B. Channel Core Produkte) ist eine sofortige Flüssigkeitsaufnahme nicht immer gegeben. Es kann an den Punkten der Flüssigkeitspenetration am Saugkern 2 zu einem sogenannten Gelblocking kommen. Dabei quellen die superabsorbierenden Partikel der Absorptionsmatrix 3 an dieser Stelle so stark auf, dass keine weitere Flüssigkeit aufgenommen werden kann. Deshalb ist eine insitu-Aufnahme durch das untersuchte Vlies durchaus als vorteilhaft anzusehen, um die Menge der freien, nicht gebundenen Flüssigkeit in den Flüssigkeitskanälen 9 zu minimieren.

Die Feinfasermischungen, die Vliese mit den Vlies-Nummern 2 und 3, weisen eine hohe Aufnahme auf, während sich die Grobfasermischungen, die Vliese mit den Vlies-Nummern 4 und 5, sich durch eine direkte Weiterleitung der Flüssigkeit auszeichnen. Ein heterogener Vliesaufbau wie die gradueller Mischung mit Grobfasern und Feinfasern, das Vlies mit der Vlies- Nummer 6, kombiniert die Vorteile der anderen beiden Lösungsansätze für einen Vliesverbund einer Oberseitenlage 5 einer Saugkernhülle 4.

Tabelle 3: Run off Daten bei 25 ml 0,9% NaCl Lösung in % und ml, sowie Flüssigkeitsaufnahme.

| Vlies-Nr. | Run off [%] | Run off [ml] | Run off Aufnahme Papier [ml] | Run off Aufnahme Vlies [ml] |
|---|---|---|---|---|
| 1 | 0,3 | 0,1 | 24,8 | 0,04 |
| 2 | 41,21 | 10,3 | 8,06 | 2,3 |
| 3 | 23,36 | 5,8 | 9,51 | 6,1 |
| 4 | 0 | 0 | 21,42 | 0,7 |
| 5 | 0 | 0 | 21,27 | 3,07 |
| 6 | 43,48 | 10,87 | 3,69 | 8,32 |

Wicking

Über das Wicking oder die Steighöhen wird das durch Kapillarkräfte im Vlies hervorgerufene Einzugsverhalten von Flüssigkeiten gegen die Schwerkraft untersucht. Die Kapillarkanäle werden durch die Stapelfasern im Vlies ausgebildet. Abhängig von den Benetzungseigenschaften der Faseroberfläche und der Größe der Kapillaren fallen die Kapillarkräfte unterschiedlich groß aus.

Je größer die gemessene Steighöhe, desto größer sind die Kapillarkräfte und der entsprechende Dochteffekt im Vliesverbund. Daraus lassen sich Rückschlüsse auf die Flüssigkeitsverteilung im Vlies ziehen.

Tabelle 4: Steighöhen in mm nach 300 Sekunden.

| Vlies-Nr. | Wicking nach 300 Sekunden [mm] |
|---|---|
| 1 | 0 |
| 2 | 64,5 |
| 3 | 139 |
| 4 | 21 |
| 5 | 19,5 |
| 6 | 104 |

Strike Through

Beim Strike Through Versuch wird eine definierte Flüssigkeitsmenge punktuell in das Vlies eingebracht und die Zeit gemessen, bis die Flüssigkeitsmenge vom Vlies aufgenommen wird. Für den Einsatz eines Vlieses im Bereich des Flüssigkeitsmanagements gilt, je kürzer die Strike Through Zeit, desto besser funktioniert das Vlies, d.h. der Transport der Flüssigkeit durch die Oberseitenlage 5 der Saugkernhülle 4.

Tabelle 5: Daten für einen dreifachen Strike Through-Versuch.

| Vlies-Nr. | Strike Through [s] |
|---|---|
| 1 | 1,6/2,4/2,2 |
| 2 | 2,3/2,6/2,6 |
| 3 | 2,9/2,6/3,5 |
| 4 | 0,86/1,08/1,25 |
| 5 | 0,72/1,38/1,4 |
| 6 | 1,14/1,89/2 |

Luftdurchlässigkeit

Durch die Messung der Luftdurchlässigkeit in l/m$^2$/s bei einem Prüfdruck von 200 Pa wird getestet, welche Luftmenge bezogen auf ein Quadratmeter Vlies innerhalb einer Sekunde durch das Vlies strömt. Vliesstoffe mit einem höheren Feinfaseranteil weisen eine geringere Luftdurchlässigkeit auf als Vliesstoffe mit einem höheren Grobfaseranteil. Das lässt sich dadurch erklären, dass Vliese mit feineren Fasern eine größere innere Oberfläche besitzen als Vliese mit gröberen Fasern. Über den Parameter der Luftdurchlässigkeit lassen sich somit Vergleiche zwischen unterschiedlichen Vliesstoffen hinsichtlich deren Dichtigkeit ziehen. Um zu verhindern, dass insbesondere die superabsorbierenden Partikel der Absorptionsmatix 3 durch das Vlies für die Saugkernhülle 4 hindurchdringen und aus dem Saugkern 2 austreten, muss die Saugkernhülle 4 eine bestimmte Dichtigkeit aufweisen, üblicherweise mindestens 3.500 l/m$^2$/s.

Tabelle 6: Daten der Luftdurchlässigkeit zur Beurteilung der Dichtigkeit der Vliesstoffe.

| Vlies-Nr. | Luftdurchlässigkeit [l/m$^2$/s] bei 200Pa |
|---|---|
| 1 | 3200 |
| 2 | 3353 |
| 3 | 1713 |
| 4 | 5300 ohne SMS-Vlies<br>2590 mit SMS-Vlies |
| 5 | 4915 ohne SMS-Vlies<br>2410 mit SMS-Vlies |
| 6 | 3000 ohne SMS-Vlies<br>2010 mit SMS-Vlies |

Für die Bewertung der gesamten Luftdurchlässigkeit von mehrlagigen Vliesen mit unterschiedlichem Dichtigkeitsgrad der Einzellagen kann eine Berechnung auf Basis der Luftdurchlässigkeit der einzelnen Vliese vorgenommen werden. Der Kehrwert der gesamten Luftdurchlässigkeit bestimmt sich dabei aus einer Addition der Kehrwerte der Luftdurchlässigkeiten der Einzellagen.

Porenvolumen

Die relative Porosität ε der in den Versuchen eingesetzten Vliesstoffe ergibt sich aus dem Porenvolumen der Vliese, die sich über folgende Formel berechnen lässt:

$$\varepsilon = 100 * \left(1 - \frac{FG}{e * \rho_{Faser}}\right)$$

Dabei ist FG das Flächengewicht in g/m$^2$, e ist die Dicke des Vliesstoffes in μm und $\rho_{Faser}$ ist die relative Dichte der Faser in g/cm$^3$.

Das Gesamtporenvolumen lässt sich entsprechend durch folgende Formel ermitteln:

$$V_0 = \varepsilon * V_{Material}$$

Tabelle 7: Berechnetes Porenvolumen.

| Vlies-Nr. | Flächengewicht [g/m$^2$] | Dicke [mm] | Fasermaterial | Feinheit [dtex] | Anteil | Porenvolumen |
|---|---|---|---|---|---|---|
| 1 | 8 | 0,14 | PP | 1,3 | 100 % | 0,940 |
| 2 | 30 | 0,49 | PP<br>CV | 1,3<br>1,7 | 27 %<br>73 % | 0,953 |
| 3 | 55 | 0,63 | PP<br>CV | 1,3<br>1,7 | 14,5 %<br>85,5 % | 0,956 |
| 4 | 68 | 1,0 | PET<br>CV<br>CoPET<br>PP | 10<br>1,7<br>4,4<br>1,3 | 35 %<br>18 %<br>35 %<br>12 % | 0,949 |

(fortgesetzt)

| Vlies-Nr. | Flächengewicht [g/m²] | Dicke [mm] | Fasermaterial | Feinheit [dtex] | Anteil | Porenvolumen |
|---|---|---|---|---|---|---|
| 5 | 68 | 1,19 | PET | 10 | 35 % | 0,957 |
|  |  |  | CV | 1,7 | 24 % |  |
|  |  |  | CoPET | 4,4 | 29 % |  |
|  |  |  | PP | 1,3 | 12 % |  |
| 6 | 68 | 0,79 | PET | 3,3 | 35 % | 0,937 |
|  |  |  | CV | 1,7 | 44 % |  |
|  |  |  | CoPET | 4,4 | 9 % |  |
|  |  |  | PP | 1,3 | 12 % |  |

Freies Volumen und Faseroberfläche

Über das freie Volumen im Vlies können Rückschlüsse über den vorhandenen Hohlraum der jeweiligen Verbundvliese gezogen werden. Das freie Volumen im Vlies bietet die Möglichkeit der Einlagerung von Flüssigkeit. Dies bedeutet, je höher das freie Volumen im Vlies ist, desto mehr Flüssigkeit kann in dem Vlies gebunden werden. Dabei kann über die Größe der Faseroberfläche im Vliesstoff auch eine Unterscheidung zwischen den Feinfaservliesen und den Grobfaservliesen der in den Versuchen eingesetzten Verbundvliese getroffen werden. Die Angaben des freien Volumens im Vlies und der Faseroberfläche im Vlies beziehen sich jeweils auf einen Quadratmeter Vlies.

Tabelle 8: Berechnete Werte des freien Volumens im Vlies und der Faseroberfläche im Vlies.

| Vlies-Nr. | Freies Volumen [mm³] | Faseroberfläche [m²] |
|---|---|---|
| 1 | 131672 | 8,6 |
| 2 | 467279 | 22,1 |
| 3 | 590923 | 27,1 |
| 4 | 1080881 | 17,2 |
| 5 | 1131150 | 16,4 |
| 6 | 768982 | 12,2 |

Ergebnisse

Aus den Ergebnissen der Versuche wird deutlich, dass es drei unterschiedliche Möglichkeiten zur Ausbildung des Stapelfaservlieses für die Transferlage 14 eines zumindest als Oberseitenlage 5 der Saugkernhülle 4 eingesetzten Verbundvlieses gibt. Die in den Versuchen eingesetzten Vliesstoffe bestehen jeweils aus einer Fixierlage 13 aus einem vorverfestigten SMS-Vlies und einer darin eingenadelten unterschiedlichen Transferlage 14 aus einem Stapelfaservlies. Für die unterschiedlichen Transferlagen 14 kann folgende Einteilung gemacht werden:

1. Feinfaserstrukturen (< 3,3 dtex) mit einem hohen Anteil an zellulosischer Faser (50-100%), nicht erfindungsgemäß,
2. Grobfaserstrukturen (> 3,3 dtex bis 12,0 dtex) mit einem moderaten Anteil an zellulosischer Faser (15-20%),
3. Kombination aus Grobfaserstruktur und Feinfaserstruktur.

Bei der erfindungsgemäßen Ausgestaltung eines Saugkerns 2 für Hygieneartikel 1 ist neben der Aufnahme der Flüssigkeit von der Saugkernhülle 3 vor allem auch ein Transfer und eine Verteilung der Flüssigkeit in Richtung der Absorptionsmatrix 4 des Saugkerns 2 gewünscht. Im Vergleich zum Stand der Technik (Vlies-Nr. 1) hilft hierbei die verbesserte Kapillarität der Verbundvliese (Vlies-Nr. 2 - 6). Die Einbindung der Stapelfasern in der Transferlage 14 in die Fixierlage 13 der Verbundvliese führt dazu, dass die Grenzflächeneffekte an der Fixierlage 13, insbesondere bei einem SMS-Vlies, minimiert werden und der Flüssigkeitstransfer von der Transferlage 13 zur Absorptionsmatrix 4 besser funktioniert. Dies lässt sich insbesondere durch den sogenannten Dochteffekt zu erklären, da die Stapelfasern der Transferlage 14 die Fixierlage 13 in Z-Richtung (senkrecht zur Oberseitenlage 5) durchdringen und kapillare Kanäle ausgebildet werden, durch die Flüssigkeit leichter in die Absorptionsmatrix 4 des Saugkerns 2 transportiert werden kann.

Auswertung

Eine reine Fixierlage (Vlies-Nr. 1), wie sie im Stand der Technik als Oberseitenlage 5 und Unterseitenlage 6 einer Saugkernhülle 3 eingesetzt wird, zeigt in den relevanten Parametern zur Beschreibung der Flüssigkeitsabsorption und -speicherung unzureichende Eigenschaften. Das Vlies-Nummer 1 weist keine Saugeigenschaft (Wicking) entgegen die Schwerkraft auf. Auch beim Run off gibt es Defizite in der Zwischenspeicherung von Flüssigkeit im Vlies. Wird die Eigenschaft der maximalen Wasseraufnahme in Betracht gezogen, wird deutlich, dass dieses sehr flache und leichtgewichtige Feinstfaservlies als Saugkernhülle 4 nur eine sehr begrenzte Fähigkeit zur Aufnahme von Flüssigkeit hat.

Das nicht erfindungsgemäße Vlies-Nummer 2 weist demgegenüber eine stark verbesserte Flüssigkeitsabsorption und Flüssigkeitsspeicherung auf, was durch den statischen Test der maximalen Wasseraufnahme mit einem Wert von über 1100 % bestätigt wird. Wird die Flüssigkeit dynamisch auf das Vlies-Nummer 2 aufgebracht, wie beim Run-off-Versuch, liefert dieser Vliesverbund eine signifikante Aufnahme der aufgebrachten Flüssigkeitsmenge von über 50 % des gesamten Volumens. Dabei gibt das Vlies-Nummer 2 gibt den größeren Teil der aufgenommenen Menge an Flüssigkeit in die darunterliegende Schicht, d.h. der Absorptionsmatrix 3 des Saugkerns 2, weiter.

Für das nicht erfindungsgemäße Vlies-Nummer 3 gilt gleiches wie für das Vlies-Nummer 2. Durch das höhere Flächengewicht ist die maximale Wasseraufnahme in Milliliter pro Quadratmeter bei diesem Vliesverbund sehr hoch, wodurch das Vlies-Nummer 3 ist in der Lage große Mengen Flüssigkeit aufzunehmen. Auch bei der dynamischen Aufgabe der Flüssigkeit im Run-off Versuch schneidet dieses Vlies-Nummer 3 sehr gut ab, da mehr als 75% der aufgebrachten Flüssigkeitsmenge absorbiert werden.

Beim Vlies-Nummer 4 bietet die Grobfaserstruktur bietet ein hohes freies Volumen pro m² Vliesoberfläche, wodurch temporär eine sehr hohe maximale Wasseraufnahme erreicht wird. Durch den reduzierten Anteil an zellulosischen Fasern kann im Run-off Versuch die gesamte Menge an aufgebrachter Flüssigkeit absorbiert werden, wobei nur ein sehr geringer Teil der absorbierten Flüssigkeitsmenge im Vlies verbleibt. Die absorbierte Flüssigkeitsmenge wird im Wesentlichen direkt und nahezu vollständig in die darunterliegende Schicht, d.h. die Absorptionsmatrix 3, weitergegeben. Beim Run-off Versuch erreicht das Vlies-Nummer 4 das beste Ergebnis von allen untersuchten Vliesaufbauten. Auch für das Vlies-Nummer 5 bietet, ähnlich wie beim Vlies-Nummer 4, die Grobfaserstruktur ein hohes freies Volumen und eine sehr hohe maximale Wasseraufnahme pro m² Vliesoberfläche. Im Run-off Versuch wird wieder die gesamte Flüssigkeitsmenge aufgenommen, jedoch wird ein geringerer Anteil der Flüssigkeit im Vlies absorbiert und nicht sofort in die darunterliegende Schicht weitergegeben.

Das Vlies-Nummer 6 ist eine Kombination aus einer Grobfaserschicht und einer Feinfaserschicht aus Stapelfasern, wodurch an der Transferlage 14, der dem Körper eines Trägers zugewandte Oberfläche, eine hohe Flüssigkeitsabsorption erreicht wird. Dabei stellt das Vlies-Nummer 6 einen besonders hohen Anteil an Hohlraum bereit, der zur Flüssigkeitsaufnahme genutzt werden kann. Die der Fixierlage 13 und schließlich der Absorptionsmatrix 4 des Saugkerns 2 zugewandte Feinfaserschicht unterstützt durch höhere Kapillarkräfte die gute Entwässerung durch die Transferlage 14, wodurch sich im Vlies-Nummer 6 eine großflächige Verteilung der Flüssigkeit ergibt.

**Bezugszeichenliste**

1   Hygieneartikel
2   Saugkern
3   Absorptionsmatrix
4   Saugkernhülle
5   Oberseitenlage
6   Unterseitenlage
7   Rand
8   Verbindungsbereich
9   Kanal
10  Deckschicht
11  Aufnahme- und Verteillage
12  Abschlusslage
13  Fixierlage
14  Transferlage
15  Spinnvlies
16  Meltblown-Vlies

**Patentansprüche**

1. Saugkern (2) für Hygieneartikel (1) mit einer Absorptionsmatrix (3), die einen großen Anteil superabsorbierender Partikel aufweist, und einer Saugkernhülle (4) zum Einhüllen der Absorptionsmatrix (3), wobei die Saugkernhülle (4) zumindest eine Oberseitenlage (5) und eine Unterseitenlage (6) aufweist und die Saugkernhülle (4) mindestens einen vom Rand des Saugkerns (2) beabstandeten Verbindungsbereich (8) aufweist in dem die Oberseitenlage (5) und die Unterseitenlage (6) miteinander verbunden sind, wobei zumindest die Oberseitenlage (5) der Saugkernhülle (4) als ein Vliesverbund mit einer Fixierlage (13) mit geringer Durchlässigkeit, um die Partikel in der Absorptionsmatrix (3) zu halten, und einer Transferlage (14) zur Aufnahme von Flüssigkeit ausgebildet ist,
   **dadurch gekennzeichnet, dass** die Fixierlage (13) als ein Spinnvlies oder ein schmelzgeblasenes Vlies und die Transferlage (14) als ein Stapelfaservlies ausgebildet ist, wobei die Stapelfasern des Stapelfaservlies Grobfasern aufweisen mit Faserfeinheiten im Bereich von 3,3 bis 12,0 dtex und einem zellulosischen Faseranteil von bis zu 45 %.

2. Saugkern (2) für Hygieneartikel (1) nach Anspruch 1,
   **dadurch gekennzeichnet, dass** die Fixierlage (13) als ein SMS-Vlies mit einem SMS-Lagenaufbau, ausgebildet ist.

3. Saugkern (2) für Hygieneartikel (1) nach Anspruch 1,
   **dadurch gekennzeichnet, dass** die Grobfasern mit einem zellulosischen Faseranteil zwischen 15 % bis 20 % ausgebildet sind.

4. Saugkern (2) für Hygieneartikel (1) nach Anspruch 1,
   **dadurch gekennzeichnet, dass** die Stapelfasern des Stapelfaservlies als eine Kombination aus Feinfasern und Grobfasern ausgebildet sind, wobei die Feinfasern Faserfeinheiten im Bereich von 1,0 bis 3,3 dtex und einen zellulosischen Faseranteil von 50 % bis 100 % und die Grobfasern Faserfeinheiten im Bereich von 3,3 bis 12,0 dtex und einen zellulosischen Faseranteil von bis zu 45 %, bevorzugt zwischen 15 % bis 20 %, aufweisen.

5. Saugkern (2) für Hygieneartikel (1) nach einem der Ansprüche 1 bis 4,
   **dadurch gekennzeichnet, dass** das Stapelfaservlies mittels eines Spunlaceprozesses hergestellt ist.

6. Saugkern (2) für Hygieneartikel (1) nach einem der Ansprüche 2 bis 5,
   **dadurch gekennzeichnet, dass** das Stapelfaservlies in das als Fixiervlies verwendete, vorverfestigte SMS-Vlies eingenadelt ist.

7. Saugkern (2) für Hygieneartikel (1) nach einem der Ansprüche 1 bis 6,
   **dadurch gekennzeichnet, dass** zumindest die Oberseitenlage (5) der Saugkernhülle (4) eine Dicke von größer als 0,35 mm aufweist, vorzugsweise eine Dicke zwischen 0,5 mm und 1,2 mm aufweist.

8. Saugkern (2) für Hygieneartikel (1) nach einem der Ansprüche 1 bis 7,
   **dadurch gekennzeichnet, dass** zumindest die Oberseitenlage (5) der Saugkernhülle (4) bei einem Prüfdruck von 200 Pa eine Luftdurchlässigkeit von maximal 3.500 l/m$^2$/s aufweist, bevorzugt von maximal 3.000 l/m$^2$/s aufweist.

9. Saugkern (2) für Hygieneartikel (1) nach einem der Ansprüche 1 bis 8,
   **dadurch gekennzeichnet, dass** zumindest die Oberseitenlage (5) der Saugkernhülle (4) bei einem Wicking-Versuch nach 300 Sekunden eine Steighöhe von mindestens 10 mm, insbesondere von mindestens 20 mm aufweist.

10. Saugkern (2) für Hygieneartikel (1) nach einem der Ansprüche 1 bis 9,
    **dadurch gekennzeichnet, dass** der Run-off-Anteil zumindest für die Oberseitenlage (5) der Saugkernhülle (4) beim einem Run-off-Versuch in Anlehnung an WSP 80.9 kleiner als 50 % ist.

11. Saugkern (2) für Hygieneartikel (1) nach einem der Ansprüche 1 bis 10,
    **dadurch gekennzeichnet, dass** die Strike-Through-Zeit für zumindest die Oberseitenlage (5) der Saugkernhülle (4) beim einem Strike-Through-Versuch kleiner als 3 Sekunden ist, bevorzugt kleiner als 1,5 Sekunden ist.

12. Hygieneartikel (1) mit einer optionalen Deckschicht (Topsheet) (10), einer Aufnahme- und Verteillage (ADL) (11), einem Saugkern (2) für Hygieneartikel (1) nach einem der Ansprüche 1 bis 11, und einer optionalen Abschlusslage (Backsheet) (12), wobei der Saugkern (2) eine Saugkernhülle (4) zum Einhüllen einer Absorptionsmatrix (3) mit einer Oberseitenlage (5) und einer Unterseitenlage (6) aufweist, die mindestens einen vom Rand (7) des Saugkerns (2) beabstandeten Verbindungsbereich (8) aufweist in dem die Oberseitenlage (5) und die Unterseitenlage (6) miteinander verbunden sind und der gegenüber der Aufnahme- und Verteillage (11) einen Kanal (9) ausbildet, wenn die Absorptionsmatrix (3) durch eine Aufnahme von Flüssigkeit aufquillt.

13. Verwendung eines Vliesverbunds mit einer Fixierlage (13) und einer Transferlage (14) als Saugkernhülle (4) eines Saugkerns (2) für Hygieneartikel (1) nach einem der Ansprüche 1 bis 11, wobei der Saugkern (2) eine Absorptionsmatrix (3) mit einem großen Anteil superabsorbierender Partikel umfasst, wobei die Saugkernhülle (4) zum Einhüllen der Absorptionsmatrix (3) ausgebildet ist, die Fixierlage (13) eine geringe Durchlässigkeit aufweist, um die Partikel in der Absorptionsmatrix (3) zu halten, und die Transferlage (14) zur Aufnahme von Flüssigkeit geeignet ist.

14. Verfahren zur Herstellung eines Vliesverbunds für eine Oberseitenlage (5) einer Saugkernhülle (4) eines Saugkerns (2) für Hygieneartikel (1) nach einem der Ansprüche 1 bis 11, mit einer Fixierlage (13) aus einem vorverfestigtes SMS- Vlies und einer Transferlage (14) aus Stapelfasern, wobei die Transferlage (14) aus Stapelfasern in das vorverfestigtes SMS-Vlies eingenadelt wird.

## Claims

1. Absorbent core (2) for sanitary products (1) with an absorption matrix (3) containing a high proportion of a super- absorbing polymer and an absorbent core cover (4) for covering the absorption matrix (3), wherein the absorbent core cover (4) comprises at least an upper side sheet (5) and a lower side sheet (6), and the absorbent core cover (4) comprises at least one connection region (8) spaced apart from the edge of the absorbent core (2), in which the upper side sheet (5) and the bottom side sheet (6) are connected to each other, wherein at least the upper side sheet (5) of the absorbent core cover (4) is formed as a fleece composite with a fixing layer (13) with low permeability to retain the particles in the absorption matrix (3) and a transfer layer (14) for absorbing liquid, **characterized in that** the fixing layer (13) is embodied as a spunbond or a meltblown fleece and the transfer layer (14) is embodied as a staple fiber fleece, wherein the staple fibers of the staple fiber fleece comprise coarse fibers with fiber finenesses in the range of 3.3 to 12.0 dtex and a cellulose fiber proportion of up to 45%.

2. Absorbent core (2) for sanitary products (1) according to claim 1, **characterized in that** the fixation layer (13) is embodied as an SMS fleece with an SMS layer structure.

3. Absorbent core (2) for sanitary products (1) according to claim 1, **characterized in that** the coarse fibers are embodied with a cellulose fiber proportion of between 15% and 20%.

4. Absorbent core (2) for sanitary products (1) according to claim 1, **characterized in that** the staple fibers of the staple fiber fleece are embodied as a combination of fine fibers and coarse fibers, wherein the fine fibers have fiber finenesses in the range from 1.0 to 3.3 dtex and a cellulose fiber proportion of 50% to 100%, and the coarse fibers have fiber finenesses in the range of 3.3 to 12.0 dtex and a cellulose fiber proportion of up to 45%, preferably between 15% and 20%.

5. Absorbent core (2) for sanitary products (1) according to one of claims 1 to 4, **characterized in that** the staple fiber fleece is produced by means of a spunlace process.

6. Absorbent core (2) for sanitary products (1) according to one of claims 2 to 5, **characterized in that the** staple fiber fleece is needled into the pre-consolidated SMS fleece used as the fixing fleece.

7. Absorbent core (2) for sanitary products (1) according to one of claims 1 to 6, **characterized in that** at least the upper side sheet (5) of the absorbent core cover (4) has a thickness greater than 0.35 mm, preferably a thickness between 0.5 mm and 1.2 mm.

8. Absorbent core (2) for sanitary products (1) according to one of claims 1 to 7, **characterized in that** at least the upper side sheet (5) of the absorbent core cover (4) has an air permeability of at most 3,500 l/m$^2$/s, preferably at most 3,000 l/m$^2$/s, at a test pressure of 200 Pa.

9. Absorbent core (2) for sanitary products (1) according to one of claims 1 to 8, **characterized in that** at least the upper side sheet (5) of the absorbent core cover (4) has a rise height of at least 10 mm, in particular at least 20 mm, after 300 seconds in a wicking test.

10. Absorbent core (2) for sanitary products (1) according to one of claims 1 to 9, **characterized in that** the run-off proportion is less than 50% for at least the upper side sheet (5) of the absorbent core cover (4) in a run-off test based on WSP 80.9.

11. Absorbent core (2) for sanitary products (1) according to one of claims 1 to 10, **characterized in that** the strike-through time for at least the upper side sheet (5) of the absorbent core cover (4) in a strike-through test is less than 3 seconds, preferably less than 1.5 seconds.

12. Sanitary product (1) with an optional topsheet (10), an absorption and distribution layer (ADL) (11), an absorbent core (2) for sanitary products (1) according to any of claims 1 to 11, and an optional backsheet (12), wherein the absorbent core (2) has an absorbent core cover (4) for enveloping an absorption matrix (3) with an upper side sheet (5) and a bottom side sheet (6), which has at least one connection area (8) spaced from the edge (7) of the absorbent core (2) featuring a connection region (8) in which the upper side sheet (5) and the bottom side sheet (6) are connected to each other and which forms a channel (9) opposite the collection and distribution layer (11) when the absorption matrix (3) swells due to the absorption of liquid.

13. Use of a fleece composite comprising a fixation layer (13) and a transfer layer (14) as an absorbent core cover (4) of an absorbent core (2) for a sanitary product (1) according to any one of claims 1 to 11, wherein the absorbent core (2) comprises an absorption matrix (3) with a high proportion of super-absorbing particles, wherein the absorbent core cover (4) is embodied to envelop the absorption matrix (3), the fixing layer (13) has a low permeability in order to retain the particles in the absorption matrix (3), and the transfer layer (14) is suitable for absorbing liquid.

14. Method for manufacturing a fleece composite for an upper side sheet (5) of an absorbent core cover (4) of an absorbent core (2) for sanitary products according to one of claims 1 to 11, with a fixation layer (13) made of a pre-consolidated SMS fleece and a transfer layer (14) made of staple fibers, wherein the transfer layer (14) made of staple fibers is needled into the pre-consolidated SMS fleece.

**Revendications**

1. Âme absorbante (2) pour articles d'hygiène (1) comportant une matrice d'absorption (3) présentant une proportion importante de particules super-absorbantes et une enveloppe d'âme absorbante (4) pour envelopper la matrice d'absorption (3), dans laquelle l'enveloppe d'âme absorbante (4) comporte au moins une couche supérieure (5) et une couche inférieure (6) et l'enveloppe d'âme absorbante (4) comporte au moins une région de liaison (8) espacée du bord de l'âme absorbante (2) dans laquelle la couche supérieure (5) et la couche inférieure (6) sont reliées entre elles, dans laquelle au moins la couche supérieure (5) de l'enveloppe d'âme absorbante (4) est réalisée comme un composite non tissé avec une couche de fixation (13) à faible perméabilité pour maintenir les particules dans la matrice d'absorption (3) et une couche de transfert (14) pour absorber un fluide,
**caractérisée en ce que** la couche de fixation (13) est réalisée comme un non-tissé filé-lié ou un non-tissé fondusoufflé et la couche de transfert (14) est réalisée comme un non-tissé à fibres discontinues, dans laquelle les fibres discontinues du non-tissé à fibres discontinues comportent des fibres grossières avec des finesses de fibre comprises dans une plage de 3,3 à 12,0 dtex et une teneur en fibres cellulosiques allant jusqu'à 45 %.

2. Âme absorbante (2) pour articles d'hygiène (1) selon la revendication 1,
**caractérisée en ce que** la couche de fixation (13) est réalisée comme un non-tissé avec une structure à triple couche Spunbond - Meltblown - Spunbond, SMS.

3. Âme absorbante (2) pour articles d'hygiène (1) selon la revendication 1,
**caractérisée en ce que** les fibres grossières sont réalisées avec une teneur en fibres cellulosiques comprise entre 15 % et 20 %.

4. Âme absorbante (2) pour articles d'hygiène (1) selon la revendication 1,
**caractérisée en ce que** les fibres discontinues du non-tissé à fibres discontinues sont réalisées comme une combinaison de fibres fines et de fibres grossières, dans laquelle les fibres fines présentent des finesses de fibres comprises dans une plage de 1,0 à 3,3 dtex et une teneur en fibres cellulosiques comprise entre 50 % et 100 %, et les fibres grossières présentent des finesses de fibres comprise dans une plage de 3,3 à 12,0 dtex et une teneur en fibres cellulosiques allant jusqu'à 45 %, de préférence comprise entre 15 % et 20 %.

**5.** Âme absorbante (2) pour articles d'hygiène (1) selon l'une des revendications 1 à 4, **caractérisée en ce que** le non-tissé à fibres discontinues est produit au moyen d'un procédé spunlace.

**6.** Âme absorbante (2) pour articles d'hygiène (1) selon l'une des revendications 2 à 5, **caractérisée en ce que** le non-tissé à fibres discontinues est aiguilleté dans le non-tissé SMS pré-consolidé utilisé comme non-tissé de fixation.

**7.** Âme absorbante (2) pour articles d'hygiène (1) selon l'une des revendications 1 à 6, **caractérisée en ce qu'**au moins la couche supérieure (5) de l'enveloppe d'âme absorbante (4) présente une épaisseur supérieure à 0,35 mm, de préférence une épaisseur comprise entre 0,5 mm et 1,2 mm.

**8.** Âme absorbante (2) pour articles d'hygiène (1) selon l'une des revendications 1 à 7, **caractérisée en ce qu'**au moins la couche supérieure (5) de l'enveloppe d'âme absorbante (4) présente une perméabilité à l'air de 3 500 l/m$^2$/s au maximum, de préférence de 3 000 l/m$^2$/s au maximum, à une pression d'essai de 200 Pa.

**9.** Âme absorbante (2) pour articles d'hygiène (1) selon l'une des revendications 1 à 8, **caractérisée en ce qu'**au moins la couche supérieure (5) de l'enveloppe d'âme absorbante (4) présente une hauteur de remontée capillaire d'au moins 10 mm, en particulier d'au moins 20 mm, après 300 secondes lors d'un test de mèche.

**10.** Âme absorbante (2) pour articles d'hygiène (1) selon l'une des revendications 1 à 9, **caractérisée en ce que** la proportion d'écoulement est inférieure à 50 % au moins pour la couche supérieure (5) de l'enveloppe d'âme absorbante (4) dans un test d'écoulement selon la norme WSP 80.9.

**11.** Âme absorbante (2) pour articles d'hygiène (1) selon l'une des revendications 1 à 10, **caractérisée en ce que** le temps de pénétration pour au moins la couche supérieure (5) de l'enveloppe d'âme absorbante (4) dans un test de pénétration est inférieur à 3 secondes, de préférence inférieur à 1,5 seconde.

**12.** Article d'hygiène (1) comportant une couche de couverture (feuille supérieure) facultative (10), une couche d'absorption et de répartition (ADL) (11), une âme absorbante (2) pour articles d'hygiène (1) selon l'une des revendications 1 à 11, et une couche finale (feuille arrière) facultative (12), dans laquelle l'âme absorbante (2) comporte une enveloppe d'âme absorbante (4) pour envelopper une matrice d'absorption (3) avec une couche supérieure (5) et une couche inférieure (6), qui comporte au moins une région de liaison (8) espacée du bord (7) de l'âme absorbante (2), dans lequel la couche supérieure (5) et la couche inférieure (6) sont reliées entre elles, et qui forme un canal (9) en face de la couche d'absorption et de distribution (11) quand la matrice d'absorption (3) gonfle en raison de l'absorption de liquide.

**13.** Utilisation d'un composite non tissé comportant une couche de fixation (13) et une couche de transfert (14) comme enveloppe d'âme absorbante (4) d'une âme absorbante (2) pour articles d'hygiène (1) selon l'une des revendications 1 à 11, dans laquelle l'âme absorbante (2) comprend une matrice d'absorption (3) avec une proportion élevée de particules super-absorbantes, dans laquelle l'enveloppe d'âme absorbante (4) est réalisée pour envelopper la matrice d'absorption (3), la couche de fixation (13) présente une faible perméabilité afin de maintenir les particules dans la matrice d'absorption (3), et la couche de transfert (14) est adaptée pour l'absorption d'un liquide.

**14.** Procédé de fabrication d'un composite non tissé pour une couche supérieure (5) d'une enveloppe d'âme absorbante (4) d'une âme absorbante (2) pour articles d'hygiène (1) selon l'une des revendications 1 à 11, comportant une couche de fixation (13) constituée d'un non-tissé SMS pré-consolidé et une couche de transfert (14) constituée de fibres discontinues, dans lequel la couche de transfert (14) de fibres discontinues est aiguilletée dans le non-tissé SMS pré-consolidé.

Fig. 1

Fig. 2

Fig. 3

Fig. 4

Fig. 5

**IN DER BESCHREIBUNG AUFGEFÜHRTE DOKUMENTE**

**In der Beschreibung aufgeführte Patentdokumente**

- EP 2717823 A1
- EP 1827335 A1

- US 2015173968 A